# EUROPEAN PATENT APPLICATION

(11) **EP 2 269 581 A1**
(43) Date of publication of application: **05.01.2011**
(21) Application number: 10175541.1
(22) Date of filing: 29.06.2007
(51) Int. Cl.: A61K 9/16, A61K 31/366

(54) **Improvements relating to biocidal compositions**

(30) Priority: 13.07.2006 GB 0613925
(62) Divisional of application: 07765734.4
(71) Applicant: Unilever PLC, A Company Registered In England And Wales under company no. 41424 of Unilever House, London EC4Y 0DY Greater London (GB); Unilever NV, 3013 AL Rotterdam (NL)
(72) Inventor: Dunclaf, David, John, Wirral Merseyside CH64 0TU (GB); Foster, Alison, Jayne, Wirral Merseyside CH63 8QB (GB); Long, James, Wirral Merseyside CH43 1TF (GB); Rannard, Steven, Paul, Liverpool Merseyside L69 7ZX (GB); Wang, Dong, Wirral Merseyside CH43 8SF (GB)
(74) Representative: Elliott, Peter William

(57) **Abstract**

The invention provides a solvent-free biocidal composition of improved efficacy comprising at least one water insoluble biocide (preferably a herbicide) and a water-soluble carrier material, wherein the water-insoluble biocide is dispersed through the carrier material in nano-disperse form having a peak diameter of the nano-disperse form below 1000nm. The invention also provides a process which comprises spray drying a solution of the biocide and a solution of a water-soluble carrier to obtain a solvent free dispersion of the biocide in the carrier, which, when dissolved in water, produces a nano-disperse biocide. The invention also provides an aqueous dispersion of a water insoluble biocide and a water-soluble carrier obtained by the dispersion of the composition of the solvent free composition in water.

## Description

### Field of the Invention

The present invention concerns improvements relating to biocidal compositions.

In particular it relates to biocidal compositions and precursors thereof which contains a water-insoluble biocidal substance.

### Background of the Invention

Biocidal agents are widely used in agriculture, sanitation and cleaning, wood and paper preservation and various other human, animal and plant health and industrial applications. The present invention is believed to be generally applicable to biocidal compositions but will be described with particular reference to antimicrobial agents, i.e. antibacterial and anti-fungal agents and also herbicides, although other and broader aspects of the invention are not intended to be excluded.

Many effective biocides have poor water solubility and various proposals have been made for how these materials can be made more effective. For example, many agricultural formulations contain water soluble salts of insoluble or poorly soluble biocides. These salts, such as alkylamine salts, are generally not as active as their acid equivalents. As an illustration of this, 2,4-Dichlorophenoxyacetic acid (2,4-D acid) is known to be more herbicidally active than the corresponding dimethylamine salt of 2, 4-D. However, the 2, 4-D acid is not soluble in water. Solvents used to formulate, 2,4-D in its acid form are known to be phytotoxic to plants and enhance herbicide volatility and subsequent drift to non-target areas.

Our co-pending international patent application PCT/GB03/03226 describes the formation of solid, porous beads comprising a three dimensional open-cell lattice of a water-soluble polymeric material. These are typically 'templated' materials formed by the removal of both water and a non-aqueous dispersed phase from a high internal phase emulsion (HIPE) which has a polymer dissolved in the aqueous phase. The macroscopic beads are formed by dropping the HIPE emulsion into a low temperature fluid such as liquid nitrogen, then freeze-drying the particles formed to remove the bulk of the aqueous phase and the dispersed phase. This leaves behind the polymer in the form of a 'skeletal' structure. The beads dissolve rapidly in water and have the remarkable property that a water-insoluble component dispersed in the dispersed phase of the emulsion prior to freezing and drying can also be dispersed in water on solution of the polymer skeleton of the beads.

WO 2005/011636 discloses a non-emulsion based spray drying process for forming 'solid amorphous dispersions' of drugs in polymers. In this method a polymer and a low-solubility drug are dissolved in a solvent and spray-dried to form dispersions in which the drug is mostly present in an amorphous form rather than in a crystalline form.

Our co-pending applications GB 0501835 (filed 28th Jan 2005, published 3^{rd} August 2006) and GB 0613925 (filed 13th July 2006) describe how materials which will form a nano-dispersion in water can be prepared, preferably by a spray-drying process. In both cases the liquid is dried above ambient temperature (above 20 Celsius), such as by spray drying, to produce particles of the structuring agent, as a carrier, with the water-insoluble materials dispersed therein. When these particles are placed in water they dissolve, forming a nano-dispersion of the water-insoluble material with particles typically below 300nm. This size scale is similar to that of virus particles, and the water-insoluble material behaves as though it were in solution.

In the process described in GB 0501835 the water insoluble material is dissolved in the solvent-phase of an emulsion. This earlier, prior published application states that the process can be applied to antimicrobial agents, for example: Triclosan™, climbazole, octapyrox, ketoconizole, phthalimoperoxyhexanoic acid (PAP) and quaternary ammonium compounds, or to insecticides, pesticides and herbicides. Our prior published GB 0501835 application also showed that fluorescer materials prepared by the method disclosed exhibited better performance than those prepared by a known freeze-drying method.

In GB 0613925 the water-insoluble materials are dissolved in a mixed solvent system and co-exist in the same phase as a water-soluble structuring agent. Our GB 0613925 application makes it clear that a Triclosan™ nano-dispersion has the additional benefit that weight for weight it is more effective than is normally expected of Triclosan™ even at very low concentrations.

In the present application the term 'ambient temperature' means 20 degrees Celsius and all percentages are percentages by weight unless otherwise specified.

### Brief Description of the Invention

We have now determined that both the emulsion-based and the single-phase method can be used to produce a water-soluble form of biocidal substances which show improved efficacy.

Accordingly, a first aspect of the present invention provides a biocidal preparation of improved efficacy comprising at least one water insoluble biocide and a water-soluble carrier material, wherein the water-insoluble biocide is dispersed through the carrier material in nano-disperse form having a peak diameter of the nano-disperse form below 1000nm.

The present invention further provides a process for improving the efficacy of a water insoluble biocide which comprises the step of spray drying a solution of the biocide and a solution of a water-soluble carrier to obtain a solvent free dispersion of the biocide in the carrier, which, when dissolved in water produces a nano-disperse biocide with a peak particle diameter of below 1000nm.

The preferred method of particle sizing for the dispersed products of the present invention employs a dynamic light scattering instrument (Nano S, manufactured by Malvern Instruments UK). Specifically, the Malvern Instruments Nano S uses a red (633nm) 4mW Helium-Neon laser to illuminate a standard optical quality UV curvette containing a suspension of material. The particle sizes quoted in this application are those obtained with that apparatus using the standard protocol.

Preferably, the peak diameter of the water-insoluble biocide is below 800nm. More preferably the peak diameter of the water-insoluble biocide is below 500nm. In a particularly preferred embodiment of the invention the peak diameter of the water-insoluble biocide is below 200nm, most preferably below 100nm.

We have determined that for smaller particle sizes, in particular for particle sizes below 40nm there is a further significant improvement in the MIC of the water insoluble biocides. This effect is not well-understood (by comparison, the thickness of a Gram-negative bacterial cell wall is around 10nm) and it may involve some specific interaction of the biocide nano-particles with the cell wall. This size range is also close to a miscellar scale and another possible explanation is that only a few molecules or in some cases a single molecule of certain biocides are needed to achieve an effect and therefore delivering a very small package of water-insoluble biocide vastly increases it's efficiency.

In the context of the present invention, "water insoluble" as applied to the biocide means that its solubility in water is less than 10g/L.

Preferably, the water insoluble biocide has solubility in water at ambient temperature (20 Celsius) of less than 5g/L preferably of less than 1g/L, especially preferably less than 120mg/L, even more preferably less than 15mg/L and most preferably less than 5mg/L. This solubility level provides the intended interpretation of what is meant by water-insoluble in the present specification.

The present invention is applicable to a broad range of biocides. Preferred water-insoluble biocides for use in the present invention are antibacterials (for example chlorophenols including Triclosan), antifungals (for example organochlorines including Chlorothalonil and imidazoles such as Ketoconazole and Propiconazole), insecticides (for example pyrethroids, including λ-cyhalothrin) and/or herbicides (for example phenol-ureas including Isoproturon). The invention is also envisaged to be applicable to acaricides, algicides, molluscicides and nematacides.

Triclosan (5-chloro-2-(2,4-dichlorophenoxy)-phenol) is an chlorophenol antibacterial used in soaps, deodorants, toothpastes, mouthwashes, and cleaning supplies and is infused in an increasing number of consumer products, such as kitchen utensils, toys, bedding, socks, and trash bags. It has a poor solubility in water of ∼17 mg/L and is a suitable antibacterial biocide for use in the present invention.

Ketoconazole (acetyl-dichlorophenyl-imidazole) is a broad spectrum imidazole antifungal agent that is used in a variety of formats to treat fungal infections. Creams, lotions and medicated shampoos are available for topical infections such as dandruff, whilst an oral tablet is used to treat systemic fungal infections. Janssen - Cilag Ltd produce a variety of ketoconazole based formulations under the trade name "Nizoral^{®}". It has a solubility of less than 0.1 mg/L.

Propiconazole (1-(2-(2,4-dichlorophenyl)-4-propyl-1-1,3-dioxolan-2-ylmethyl)-1H-1,2,4-tri azole) is another broad spectrum antifugal agent. Propiconazole is predominantly used in antifungal agrochemical formulations such as "Tilt^{®}" manufactured by Ciba. It has a solubility of around 100mg/L.

Azoxystrobin (methyl (E)-2-{2-[6-(2-cyanophenoxy)pyrimidin-4-yloxy] phenyl}-3-methoxyacrylate) is a systemic, broad-spectrum strobilurin fungicide with activity against the four major groups of plant pathogenic fungi including *Ascomcetes* (eg powdery mildews), *Basidiomycetes* (eg rusts), *Deutoromycetes* (eg rice blast) and *Oomycetes* (eg downy mildew). Other strobilurins are azoxystrobin, kresoximmethyl, picoxystrobin, fluoxastrobin, oryzastrobin, dimoxystrobin, pyraclostrobin and trifloxystrobin. Azoxystrobin has a very poor solubility in water of ∼6mg/L

Chlorothalonil (2,4,5,6-tetrachloroisophthalonitrile) is a broad-spectrum organo-chlorine fungicide used to control fungi that threaten vegetables, trees, small fruits, turf, ornamentals, and other agricultural crops. It has an exceptionally low solubility in water of ∼0.6 mg/L.

Ketoconazole, propiconazole, azoxystrobin and chlorothalonil are each suitable antifungal biocides for use in the present invention.

Isoproturon (3-(4-isopropylphenyl)-1,1-dimethylurea) is a common herbicide with a low solubility in water (∼65mg/L) and is widely used for the control of broad leaved weeds that grow in various annual grasses. It is a suitable herbicide for use in the present invention.

λ-cyhalothrin, is a suitable insecticide for use in the present invention and has an aqueous solubility of 0.005mg/L

As noted above, it is believed that reduction of the particle size in the eventual nano-dispersion has significant advantages in improving the effectiveness of the otherwise water-insoluble material. This is believed to be particularly advantageous where an significantly improved bio-availability is sought, or, in similar applications where high local concentrations of the material are to be avoided. Moreover it is believed that nano-dispersions with a small particle size are more stable than those with a larger particle size.

Preferred carrier materials are selected from the group consisting of water-soluble inorganic materials, surfactants, polymers, sugars and mixtures thereof.

A further aspect of the present invention provides an aqueous dispersion of a water insoluble biocide and a water-soluble carrier material, wherein the biocide is in nano-disperse form having a peak diameter of the nano-disperse form below 1000nm, preferably below 800nm, more preferably below 500nm, even more preferably below 200nm and especially below 100nm. As noted above, it is particularly advantageous when the particle size of the biocide is below 40nm.

A further aspect of the present invention provides a method for preparing a biocide composition comprising a water insoluble biocide and a water-soluble carrier, which comprises the steps of:
a) forming an emulsion comprising:
   i) a solution of the biocide in at least one water-immiscible solvent for the same, and
   ii) an aqueous solution of the carrier, and,
b) drying the emulsion to remove water and the water-immiscible solvent to obtain a substantially solvent-free nano-dispersion of the biocide in the carrier

For convenience, this class of method is referred to herein as the "emulsion" method.

A further aspect of the present invention provides a method for preparing a biocide composition comprising a water insoluble biocide and a water-soluble carrier which comprises the steps of:
a) providing a mixture comprising:
   i) at least one non-aqueous solvent,
   ii) optionally, water,
   iii) a water-soluble carrier material soluble in the mixture of (i) and (ii) and
   iv) a water-insoluble biocide agent which is soluble in the mixture of (i) and (ii), and,
b) drying said mixture to remove the non-aqueous solvent and any water present to obtain a substantially solvent-free a nano-dispersion of the biocide in the carrier.

For convenience, this class of method is referred to herein as the "single-phase" method.

In the context of the present invention substantially solvent free means that the free solvent content of the product is less than 15%wt, preferably below 10%wt and more preferably below 5%wt.

In the context of the present invention it is essential that both the carrier material and the biocide are essentially fully dissolved in their respective solvents prior to the drying step. It is not within the ambit of the present specification to teach the drying of slurries. For the avoidance of any doubt, it is therefore the case that the solids content of the emulsion or the mixture is such that over 90%wt, preferably over 95%, and more preferably over 98% of the soluble materials present is in solution prior to the drying step.

In relation to the methods mentioned above, the preferred biocide and the preferred carrier materials are as described above and as elaborated on in further detail below. Similarly the preferred physical characteristics of the material are as described above.

The "single phase" method where both the biocide and the carrier material are dissolved in a phase comprising at least one non-aqueous solvent (and optional water) is preferred. This is believed to give a smaller particle size for the nano-disperse biocide. Preferably, drying simultaneously removes essentially all solvents and, more preferably, is accomplished by spray drying at above ambient temperature.

A further aspect of the present invention provides a method for the preparation of a biocide composition for use in the prophylaxis or treatment of infections or infestations which comprises the step of preparing a composition according to the present invention. Preferably, the method is one in which the particle size of the water-insoluble biocide is reduced to below 100 nm, more preferably below 40nm. Such compositions are suitable for use in methods of medical treatment.

A still further aspect of the present invention provides for the treatment of a substrate other than a medical treatment which comprises the step of contacting the substrate with a composition according to the present invention. Such a method might comprise, for example, a method for preserving wood or other materials of natural origin.

### Detailed Description of the Invention

Various preferred features and embodiments of the present invention are described in further detail below.

### Biocide:

As noted above, the present invention is applicable to a broad range of water-insoluble biocides. Preferred biocides are non-animal biocides, particularly fungicides, bactericides and herbicides. Preferably these biocides have solubility in water of less than 120 mg/L and more preferably less than 15mg/L.

In the context of the present invention the term biocide also includes biostats. For example propiconazole is "fungistatic" rather than "fungicidal" as its mode of action involves inhibition of cell mitosis, rather than causing cell death.

Some known water-insoluble herbicides are listed in US Patent 6849577 and include diuron, linuron, sulfometuron, chlorsulphuron, metsulfuron, chlorimuron, atrazine, simazine, quizalofop, butroxydim, nicosulfuron, primsulfuron, bensulfuron, ametryn, pendimethalin, isoproturon, chlortoluron, diflufenican, mesotrione, aclonifen, flurochloridone, oxyfluorfen, isoxaflutole, imazamox and thifensulfuron.

Other suitable herbicides include trifluralin, fluoroxypyr, phenmedipham, fenoxaprop-P-ethyl, acetochlor, alachlor, triallate and propanil.

The present invention, in its broadest sense, does not depend critically on the nature of the water-insoluble herbicide. The invention is suitable for application with the insoluble form of those herbicides, as mentioned above, which are currently used in salt form. These include glyphosphate (N-phosphonomethylglycine), which is commonly used in the form of its water-soluble salts such as trimethylsulphonium, isopropylamine, sodium, or ammonium salts, fomesafen which is commonly used in the form of its water-soluble sodium salt, glufosinate which is commonly used in the form of its water-soluble ammonium salt, paraquat dichloride and bentazone which is commonly used in the form of its water-soluble sodium salt.

Some known water-insoluble fungicides are disclosed in, for example, US Patents 6355675 and 6113936 and include benzimidazole compounds such as benomyl, carbendazim, thiabendazole and thiophanate-methyl; phenylcarbamate compounds such as diethofencarb; dicarboxyimide compounds such as procymidone, iprodione and vinclozolin; azole compounds such as diniconazole, epoxyconazole, tebuconazole, difenoconazole, cyproconazole, flusilazole, flutriafol and triadimefon; acylalanine compounds such as metalaxyl; carboxyamide compounds such as furametpyr, mepronil, flutolanil and tolyfluanid; organophosphate compounds such as tolclofos-methyl, fosetyl aluminum and pyrazophos; anilinopyrimidine compounds such as pyrimethanil, mepanipyrim and cyprodinil; cyanopyrrrole compounds such as fludioxonil and fenpiclonil; antibiotics such as blasticidin-S, kasugamycin, polyoxin and validamycin; methoxyacrylate compounds such as azoxystrobin, kresoximmethyl and metominostrobin; chlorothalonil; manzeb; captan; folpet;;; tricyclazole; pyroquilon; probenazole; phthalide; cymoxanil; dimethomorph; S-methylbenzo[1,2,3]thiadiazol-7-carbothioate; famoxadone; oxolinic acid; fluaziname; ferimzone; chlobenthiazone; isovaledione; tetrachloroisophthalonitrile; thiophthalimideoxybisphenoxyarsine; 3-iodo-2-propylbutylcarbamate;; parahydroxy benzoic ester;. Others such as fenpropimorph (morpholine based) and thiram (dithiocarbamate) are also believed suitable.

Preferred fungicides include those of the polyene, imidazole and triazole types.

Particular preferred polyenes include Amphotericin, Nystatin and mixtures thereof.

Preferred imidazoles include: Bifonazole, Butoconazole, Clotrimazole, Econazole, Fenticonazole, Isoconazole, Ketoconazole, Metronidazole, Oxiconazole, Sertaconazole, Sulconazole, Tioconazole, Miconazole and mixtures thereof.

Preferred triazole types include: Fluconazole, Itraconazole, Posaconazole, propiconazole, Ravuconazole, tebuconazole, Terconazole, Voriconazole and mixtures thereof.

Other suitable antifungal biocides for use in the present invention include: Amorolfine, Anidulafungin, Butenafine, Naftifine, Caspofungin, Ciclopirox, Flucytosine, Griseofulvin, Haloprogin, Micafungin, Parabens, Salicylic acid, Terbinafine, Thiabenazole, Tolnaflate, Undecylenic acid and mixtures thereof.

Water insoluble insecticides include cypermethrin, lambda-cyhalothrin, esfenvalerate, malathion, and chlorpyrifos.

### Water-Dispersible Form:

The present invention provides a method for obtaining a rapidly dispersible form of an otherwise essentially water-insoluble material. This is prepared by forming an at least partially non-aqueous intermediate emulsion or solution in which both a water-soluble carrier material and the water-insoluble biocide are dissolved. On removal of solvents the insoluble biocide is left dispersed through the water-soluble carrier material. Suitable carrier materials are described in further detail below.

The most preferred method for drying of the intermediate emulsion or solution is one which produces a powder directly, such as spray drying. Spray drying is particularly effective at removing both the non-aqueous volatile components and any water present to leave the carrier and the 'payload' material behind in a powder form. The drying step is described in further detail below.

The structure of the material obtained after the drying step is not well understood. It is believed that the resulting dry powder materials are not encapsulates, as discrete macroscopic bodies of the water-insoluble materials are not present in the dry product. Neither are the dry materials 'dry emulsions' as little or none of the volatile solvent comprising the 'oil' phase of the emulsion remains after the drying step. On addition of water to the dry product the emulsion is not reformed, as it would be with a 'dry emulsion'. It is also believed that the compositions are not so-called solid solutions, as with the present invention the ratios of components present can be varied without loss of the benefits. Also from Xray and DSC studies, it is believed that the compositions of the invention are not solid solutions, but comprise nano-scale, phase-separated mixtures.

Preferably, the compositions produced after the drying step will comprise the biocide and the carrier in a weight ratio of from 1:500 to 1:1 as biocide:carrier, with 1:100 to 1:1 being preferred. Typical levels of around 10-30%wt water-insoluble biocide and 90-70% carrier can be obtained by spray drying. Levels of biocide below 40%, more preferably below 30%wt and most preferably below 25%wt are preferred as they show the improved MIC as discussed above.

### "Emulsion" Preparation Method:

In one preferred method according to the invention the solvent for the water-insoluble biocide is not miscible with water. On admixture with water it therefore can form an emulsion.

Preferably, the non-aqueous phase comprises from about 10 % to about 95 % v/v of the emulsion, more preferably from about 20 % to about 68 % v/v.

The emulsions are typically prepared under conditions which are well known to those skilled in the art, for example, by using a magnetic stirring bar, a homogeniser, or a rotational mechanical stirrer. The emulsions need not be particularly stable, provided that they do not undergo extensive phase separation prior to drying.

Homogenisation using a high-shear mixing device is a particularly preferred way to make an emulsion in which the aqueous phase is the continuous phase. It is believed that this avoidance of coarse emulsion and reduction of the droplet size of the dispersed phase of the emulsion, results in an improved dispersion of the biocide in the dry product. In a preferred method according to the invention a water-continuous emulsion is prepared with an average dispersed-phase droplet size (using the Malvern peak intensity) of between 500nm and 5000nm. We have found that an 'Ultra-Turrux' T25 type laboratory homogenizer (or equivalent) gives a suitable emulsion when operated for more than a minute at above 10,000 rpm.

There is a directional relation between the emulsion droplet size and the size of the particles of the biocide, which can be detected after dispersion of the materials of the invention in aqueous solution. We have determined that an increase in the speed of homogenization for precursor emulsions can decrease final particle size after redissolution.

It is believed that the re-dissolved particle size can be reduced by nearly one half when the homogenization speed increased from 13,500 rpm to 21,500 rpm. The homogenization time is also believed to play a role in controlling re-dissolved particle size. The particle size again decreases with increase in the homogenization time, and the particle size distribution become broader at the same time. Such intensive mixing is not an essential step in the method of the present invention but it is advantageous.

Sonication is also a particularly preferred way of reducing the droplet size for emulsion systems. We have found that a Hert Systems Sonicator XL operated at level 10 for two minutes is suitable.

### "Single Phase" Preparation Method:

In an alternative method according to the present invention both the carrier and the biocide are soluble in either a non-aqueous solvent or in a mixture of water and a non-aqueous solvent. Both here and elsewhere in the specification the non-aqueous solvent can be a mixture of non-aqueous solvents. In this case the feedstock of the drying step comprises a single phase material in which both the water-soluble carrier and the water-insoluble biocide are dissolved. It is also possible for this feedstock to be an emulsion, provided that both the carrier and the biocide are dissolved in the same phase.

The 'single-phase' method is generally believed to give a better nano-dispersion with a smaller particle size than the emulsion method. As noted above, the smaller particle sizes give enhanced biocidal effects.

It is believed that ratios of components which decrease the relative concentration of the biocide to the solvents and/or the carrier give a smaller particle size.

### Drying:

Spray drying, the most preferred method of drying the emulsion or solution, is well known to those versed in the art. In the case of the present invention some care must be taken due to the presence of a volatile non-aqueous solvent in the material being dried. In order to reduce the risk of explosion when a flammable solvent is being used, an inert gas, for example nitrogen, can be employed as the drying medium in a so-called closed spray-drying system. The solvent can be recovered and re-used.

We have found that the 'Buchi' B-290 type laboratory spray drying apparatus is suitable for the performance of the present invention.

It is preferable that the drying temperature should be at or above 100 Celsius, preferably above 120 Celsius and most preferably above 140 Celsius. Elevated drying temperatures have been found to give smaller particles in the re-dissolved nano-disperse material.

Freeze drying can also be used. It is preferred to use a non-aqueous solvent with a melting point above -120 Celsius, preferably above -80 Celsius. Chloroform is a particularly preferred solvent due to it physical characteristics. It a relatively high melting point (approx. -63.5°C). Freeze drying can be employed both with the emulsion method and the single phase method.

### Carrier Material:

The carrier material is water soluble, which includes the formation of structured aqueous phases as well as true ionic solution of molecularly mono-disperse species. The carrier material preferably comprises an inorganic material, surfactant, a polymer or may be a mixture of two or more of these.

It is envisaged that other non-polymeric, organic, water-soluble materials such as sugars can be used as the carrier. However the carrier materials specifically mentioned herein are preferred.

Suitable carrier materials (referred to herein as 'water soluble carrier materials') include preferred water-soluble polymers, preferred water-soluble surfactants and preferred water-soluble inorganic materials. Particularly preferred materials are solids, as opposed to soft solids or semi-solids at ambient temperature such that good powder properties are obtained in the spray-dried product.

The particular choice of carrier material will depend on the proposed end-use of the composition and carriers should be selected such that they are not detrimentally reactive towards the biocide and compatible with the proposed use. The carrier can also have an activity in its own right or contain water soluble materials which have such an activity. For example, in agricultural applications of the present invention, the carrier may comprise materials having an agrochemical activity.

### Preferred polymeric carrier materials:

Examples of suitable water-soluble polymeric carrier materials include:
(a) natural polymers (for example naturally occurring gums such as guar gum, alginate, locust bean gum or a polysaccharide such as dextran;
(b) cellulose derivatives for example xanthan gum, xyloglucan, cellulose acetate, methylcellulose, methyl-ethylcellulose, hydroxy-ethylcellulose, hydroxyethylmethyl-cellulose, hydroxy-propylcellulose, hydroxy-propylmethylcellulose, hydroxy-propylbutylcellulose, ethylhydroxy-ethylcellulose, carboxy-methylcellulose and its salts (eg the sodium salt - SCMC), or carboxy-methylhydroxyethylcellulose and its salts (for example the sodium salt);
(c) homopolymers of or copolymers prepared from two or more monomers selected from: vinyl alcohol, acrylic acid, methacrylic acid, acrylamide, methacrylamide, acrylamide methylpropane sulphonates, aminoalkylacrylates, aminoalkyl-methacrylates, hydroxyethylacrylate, hydroxyethylmethylacrylate, vinyl pyrrolidone, vinyl imidazole, vinyl amines, vinyl pyridine, ethyleneglycol and other alkylene glycols, ethylene oxide and other alkylene oxides, ethyleneimine, styrenesulphonates, ethyleneglycolacrylates and ethyleneglycol methacrylate
(d) cyclodextrins, for example beta-cyclodextrin
(e) mixtures thereof

When the polymeric material is a copolymer it may be a statistical copolymer (heretofore also known as a random copolymer), a block copolymer, a graft copolymer or a hyperbranched copolymer. Co-monomers other than those listed above may also be included in addition to those listed if their presence does not destroy the water soluble or water dispersible nature of the resulting polymeric material.

Examples of suitable and preferred homopolymers include poly-vinylalcohol, poly-acrylic acid, poly-methacrylic acid, poly-acrylamides (such as poly-N-isopropylacrylamide), polymethacrylamide; poly-acrylamines, poly-methyl-acrylamines, (such as polydimethylaminoethylmethacrylate and poly-N-morpholinoethylmethacrylate), polyvinylpyrrolidone, polystyrenesulphonate, polyvinylimidazole, polyvinylpyridine, poly-2-ethyl-oxazoline poly-ethyleneimine and ethoxylated derivatives thereof.

Polyethylene glycol (PEG), polyvinylpyrrolidone (PVP), poly(2-ethyl-2-oxazaline), polyvinyl alcohol (PVA) hydroxypropyl cellulose and hydroxypropyl-methyl cellulose (HPMC) and alginates are preferred polymeric carrier materials.

### Preferred surfactant carrier materials:

Where the carrier material is a surfactant, the surfactant may be non-ionic, anionic, cationic, amphoteric or zwitterionic.

Examples of suitable non-ionic surfactants include ethoxylated triglycerides; fatty alcohol ethoxylates; alkylphenol ethoxylates; fatty acid ethoxylates; fatty amide ethoxylates; fatty amine ethoxylates; sorbitan alkanoates; ethylated sorbitan alkanoates; alkyl ethoxylates; Pluronics™; alkyl polyglucosides; stearol ethoxylates; alkyl polyglycosides.

Examples of suitable anionic surfactants include alkylether sulfates; alkylether carboxylates; alkylbenzene sulfonates; alkylether phosphates; dialkyl sulfosuccinates; sarcosinates; alkyl sulfonates; soaps; alkyl sulfates; alkyl carboxylates; alkyl phosphates; paraffin sulfonates; secondary n-alkane sulfonates; alpha-olefin sulfonates; isethionate sulfonates.

Examples of suitable cationic surfactants include fatty amine salts; fatty diamine salts; quaternary ammonium compounds; phosphonium surfactants; sulfonium surfactants; sulfonxonium surfactants.

Examples of suitable zwitterionic surfactants include N-alkyl derivatives of amino acids (such as glycine, betaine, aminopropionic acid); imidazoline surfactants; amine oxides; amidobetaines.

Mixtures of surfactants may be used. In such mixtures there may be individual components which are liquid, provided that the carrier material overall, is a solid.

Alkoxyayed nonionic's (especially the PEG/PPG eg Pluronic™ materials and/or the PEG/alcohol nonionics), phenol-ethoxylates (especially TRITON™ materials), alkyl sulphonates (especially SDS), ether-sulphates (including SLES), ester surfactants (preferably sorbitan esters of the Span™ and Tween™ types) and cationics (especially cetyltrimethylammonium bromide - CTAB) are particularly preferred as surfactant carrier materials.

Surfactant carrier materials are particularly suitable for embodiments of the invention in which the re-dispersed particle size in water is below 100nm, and particularly below 40nm.

### Preferred inorganic carrier materials:

The carrier material can also be a water-soluble inorganic material which is neither a surfactant not a polymer. Simple organic salts have been found suitable, particularly in admixture with polymeric and/or surfactant carrier materials as described above. Suitable salts include carbonate, bicarbonates, halides, sulphates, nitrates and acetates, particularly soluble salts of sodium, potassium and magnesium. Preferred materials include, sodium carbonate, sodium bicarbonate and sodium sulphate. These materials have the advantage that they are cheap and physiologically acceptable. They are also relatively inert as well as compatible with many materials found in household and pharmaceutical products.

Mixtures of carrier materials are advantageous. Preferred mixtures include combinations of inorganic salts and surfactants and polymers and surfactants.

Particularly preferred mixtures include combinations of surfactants and polymers. Which include at least one of:
a) Polyethylene glycol (PEG), polyvinylpyrrolidone (PVP), poly(2-ethyl-2-oxazaline), polyvinyl alcohol (PVA) hydroxypropyl cellulose and hydroxypropyl-methyl cellulose (HPMC) and alginates and, at least one of;
b) alkoxylated nonionic's (especially the PEG/PPG Pluronic™ materials), phenol-ethoxylates (especially TRITON™ materials), alkyl sulphonates (especially SDS), ether-sulphates (including SLES), ester surfactants (preferably sorbitan esters of the Span™ and Tween™ types) and cationics (especially cetyltrimethylammonium bromide - CTAB)

The carrier material can also be a water-soluble small organic material which is neither a surfactant , a polymer nor an inorganic carrier material. Simple organic sugars have been found to be suitable, particularly in admixture with a polymeric and/or surfactant carrier material as described above. Suitable small organic materials include mannitol, polydextrose, xylitol and inulin etc.

In preferred forms of the invention the level of surfactant carrier is such that at least 50% of the total carrier is surfactant. Mixtures having a majority of surfactant present over the other carriers exhibit better biocidal effects.

### Non-aqueous solvent:

The compositions of the invention comprise a volatile, non-aqueous solvent. As noted above this can be a mixture of solvents. This may either be miscible with such other solvents (including water) which may be present in the premix before drying or, together with those solvents may form an emulsion.

In one alternative form of the invention a non-aqueous solvent is employed in which can form a single phase with water in the presence of the biocide, and the carrier. Preferred solvents for these embodiments are polar, protic or aprotic solvents. Generally preferred solvents have a dipole moment greater than 1 and a dielectric constant greater than 4.5.

Particularly preferred solvents are selected from the group consisting of haloforms (preferably di-chloromethane, chloroform), lower (C1-C10) alcohols (preferably methanol, ethanol, isopropanol, isobutanol), organic acids (preferably formic acid, acetic acid), amides (preferably formamide, N,N-dimethylformamide), nitriles (preferably aceto-nitrile), esters (preferably ethyl acetate) aldehydes and ketones (preferably methyl ethyl ketone, acetone), and other water miscible species comprising hetroatom bond with a suitably large dipole (preferably tetrahydrofuran, dialkylsulphoxide). Mixtures of the aforementioned may also be employed.

In another alternative form of the invention the non-aqueous solvent is not miscible with water and forms an emulsion.

The non-aqueous phase of the emulsion is preferably selected from one or more from the following group of volatile organic solvents:
- alkanes, such as heptane, n-hexane, isooctane, dodecane, decane;
- cyclic hydrocarbons, such as toluene, xylene, cyclohexane;
- halogenated alkanes ,such as dichloromethane, dichoroethane, trichloromethane (chloroform), fluorotrichloromethane and tetrachloroethane;
- esters such as ethyl acetate;
- ketones such as 2-butanone;
- ethers such as diethyl ether;
- volatile cyclic silicones such as either linear or cyclomethicones containing from 4 to 6 silicon units. Suitable examples include DC245 and DC345, both of which are available from Dow Corning Inc.

Preferred solvents include dichloromethane, chloroform, ethanol, acetone and dimethyl sulphoxide.

Preferred non-aqueous solvents, whether miscible or not have a boiling point of less than 150 Celsius and, more preferably, have a boiling point of less than 100 Celsius, so as to facilitate drying, particularly spray-drying under practical conditions and without use of specialised equipment. Preferably they are non-flammable, or have a flash point above the temperatures encountered in the method of the invention.

Preferably, the non-aqueous solvent comprises from about 10 % to about 95 % v/v of any emulsion formed, more preferably from about 20 % to about 80 % v/v. In the single phase method the level of solvent is preferably 20-100%v/v.

Particularly preferred solvents are alcohols, particularly ethanol and halogenated solvents, more preferably chlorine-containing solvents, most preferably solvents selected from (di- or tri- chloromethane).

### Optional Cosurfactant:

In addition to the non-aqueous solvent an optional co-surfactant may be employed in the composition prior to the drying step. We have determined that the addition of a relatively small quantity of a volatile cosurfactant reduced the particle diameter of the material produced. This can have a significant impact on particle volume. For example, reduction from 297nm to 252nm corresponds to a particle size reduction of approximately 40%. Thus, the addition of a small quantity of co-surfactant offers a simple and inexpensive method for reducing the particle size of materials according to the present invention without changing the final product formulation.

Preferred co-surfactants are short chain alcohols or amine with a boiling point of <220°C.

Preferred co-surfactants are linear alcohols. Preferred co-surfactants are primary alcohols and amines. Particularly preferred co-surfactants are selected from the group consisting of the 3-6 carbon alcohols. Suitable alcohol co-surfactants include n-propanol, n-butanol, n-pentanol, n-hexanol, hexylamine and mixtures thereof.

Preferably the co-surfactant is present in a quantity (by volume) less than the solvent preferably the volume ratio between the solvent and the co-surfactant falls in the range 100:40 to 100:2, more preferably 100:30 to 100:5.

### Preferred Drying Feedstocks:

The drying feed-stocks used in the present invention are either emulsions or solutions which preferably do not contain solid matter and in particular preferably do not contain any undissolved biocide.

It is particularly preferable that the level of the biocide in the composition should be such that the loading in the dried composition is below 40%wt, and more preferably below 30%wt. Such compositions have the advantages of a small particle size and high effectiveness as discussed above.

Typical spray drying feedstocks comprise:
a) a surfactant,
b) at least one lower alcohol,
c) more than 0.1% of at least one water-insoluble biocide dissolved in the feedstock,
d) a polymer, and,
e) optional water

Preferred spray-drying feedstocks comprise:
a) at least one non-aqueous solvent selected from dichloromethane, chloroform, ethanol, acetone, and mixtures thereof,
b) a surfactant selected from alkoxylated nonionic's (especially the PEG/PPG Pluronic™ materials), phenol-ethoxylates (especially TRITON™ materials), alkyl sulphonates (especially SDS), ether-sulphates (including SLES), ester surfactants (preferably sorbitan esters of the Span™ and Tween™ types) and cationics (especially cetyltrimethylammonium bromide - CTAB), and mixtures thereof,
c) more than 0.1% of at least one water-insoluble biocidal agent,
d) a polymer selected from Polyethylene glycol (PEG), Polyvinyl alcohol (PVA), polyvinyl-pyrrolidone (PVP), hydroxypropyl cellulose and hydroxypropyl-methyl cellulose (HPMC) , alginates and mixtures thereof, and
e) optionally water.

### Water Dispersed Form:

Solid compositions according to the present invention (preferably those obtained by spray drying) are suitable for use in the treatment of an infestation. They can be used "as-is" in the solid form, but it is preferred that they are dissolved in water prior to use.

On admixture of the water-soluble carrier material with water, the carrier dissolves and the water-insoluble biocide is dispersed through the water in sufficiently fine form that it behaves like a soluble material in many respects. The particle size of the water-insoluble materials in the dry product is preferably such that, on solution in water the water-insoluble materials have a particle size of less than 1 micron as determined by the Malvern method described herein. Preferably the determined particle size is less than 800nm, more preferably less than 500nm. In typical embodiments of the invention the particle size is in the range 250-50nm and is most preferably in the range 200-75nm. For comparative purposes, the broader range is analogous to the size of a virus particle (which typically range from 450-20nm). Diameters of less than 200nm are most preferred. It is believed that there is no significant reduction of particle size for the biocidal agent on dispersion of the solid form in water.

Very small particle sizes of as low as 4nm can be obtained by the method of the invention. In the size range 4-40nm the compositions of the invention show a further improvement in efficacy.

By applying the present invention significant levels of 'water-insoluble' materials can be brought into a state which is in many respects equivalent to true solution. When the dry product is dissolved in water it is also possible to achieve visually clear, transparent "solutions" comprising more than 0.1%, preferably more than 0,5% and more preferably more than 1% of the 'water-insoluble' material. For translucent and opaque 'solutions' higher levels of nano-disperse material can be tolerated.

Advantageously, these "solutions" can be made up using water and need not contain other solvents. This means that "insoluble" biocides can be delivered by aqueous spraying, washing or infusion, without the target of the biocide being exposed to solvents.

It is envisaged that the solution form will be a form suitable for use either 'as is' or following further dilution or admixture with other components.

The present invention therefore also relates to a method for the delivery of a water-insoluble biocide which comprises the steps of:
a) dissolving in water a nano-dispersion of a water-insoluble biocide in a water soluble carrier material, wherein the water-insoluble biocide is dispersed in the carrier material in particles having an average particle size below one micron,
b) optionally, adding other components to the dispersion, and,
c) treating a substrate with the aqueous nano-dispersion of the biocide.

The substrate can be the subject to be treated directly with the biocide (such as a plant, where the object is the eradication of, for example, fungi, or a wooden object requiring rot prevention treatment) or can be associated with the subject (such as bedding or soil). Preferred substrates are selected from a plant (or part thereof, including seeds, bulbs, fruits, roots, leaves), soil, an animal, bedding for animals, fodder or an article manufactured from a plant or an animal. Preferred treatment methods include spraying, dipping and washing.

In order that the present invention may be further understood and carried forth into practice it is further described below with reference to non-limiting examples.

### Examples:

A method of particle sizing for the dispersed products of the present invention used in the following examples employs a dynamic light scattering instrument (Nano S, manufactured by Malvern Instruments UK). Specifically, the Malvern Instruments Nano S uses a red (633nm) 4mW Helium-Neon laser to illuminate a standard optical quality UV curvette containing a suspension of material.

### Example 1

### Nano-dispersions of Chlorothalonil (water-insoluble fungicide) from a single phase chloroform solution stabilised with PVP and PEG-PPG-PEG

A solution was prepared of the following:

| *Composition* | |
|---|---|
| Chlorothalonil | 0.2g (10wt.%) |
| PEG-PPG-PEG | 0.4g (20wt.%) |
| PVP (90kDa) | 1.4g (70wt.%) |
| Chloroform | 40ml |

At these concentrations, the solid components were readily soluble in the chloroform at the measured room temperature (21.5°C).

The solution was spray dried using a Buchi B-290 ™ bench top spray-dryer, operated in a negative pressure mode. Air drawn from the lab was used as the drying medium and the operating conditions were as follows:

| | |
|---|---|
| Pump rate | 10% (3.6ml/min) |
| Inlet temperature | 105°C |
| Aspiration | 100% |
| N₂ flow (atomisation) Max | (approx. 55L/hr) |

A dry white powder was obtained. This material was redispersed in demineralised water at a concentration of 10mg/ml (1.0wt%, 0.1wt% chlorothalonil). This produced an opaque white dispersion. At this concentration, the material was relatively slow to disperse (approx. 5 minutes).

The resulting solution had the following properties:

| | |
|---|---|
| Viscosity | 1.95cP |
| Particle size | **437nm** (diameter) |
| Standard deviation | ± 25.5nm |
| PdI | 0.385 |

### Example 2

### Nano-dispersion of Chlorothalonil (water-insoluble fungicide) from a single phase chloroform solution stabilised with PVP and PEG-PPG-PEG

| *Composition* | |
|---|---|
| Chlorothalonil | 0.2g (10wt.%) |
| PEG-PPG-PEG | 0.4g (20wt.%) |
| PVP (55kDa) | 1.4g (70wt.%) |
| Chloroform | 40ml |

At these concentrations, the solid components are readily soluble in chloroform at room temperature (21.5°C).

### Drying

The solution was spray dried using a Buchi B-290 bench top spray dryer, operated in a negative pressure mode. Air drawn from the lab was used as the drying medium.

| | |
|---|---|
| Pump rate | 15% (5.4ml/min) |
| Inlet temperature | 90°C |
| Aspiration | 100% |
| N₂ flow (atomisation) Max | (approx. 55L/hr) |

### Product

A dry white powder was obtained. This material was redispersed in demineralised water at a concentration of 1mg/ml (0.1wt%, 0.01wt% chlorothalonil). This produced an opaque white dispersion. At this concentration, the material was considerably quicker (than example 1) to disperse (less than 30 seconds).

| | |
|---|---|
| Viscosity | 1.0cP |
| Particle size | **452nm** (diameter) |
| Standard deviation | ± 5.72nm |
| PdI | 0.181 |

### Example 3

### Nano-dispersion of Chlorothalonil (water-insoluble fungicide) from a single phase chloroform solution stabilised with PVP and PEG-PPG-PEG

| *Composition* | |
|---|---|
| Chlorothalonil | 0.05g (10wt.%) |
| PEG-PPG-PEG | 0.1g (20wt.%) |
| PVP (55kDa) | 0.35g (70wt.%) |
| Chloroform | 30ml |

At these concentrations, the solid components are readily soluble in chloroform at room temperature (21.5°C).

### Drying

The solution was spray dried using a Buchi B-290 bench top spray dryer, operated in a negative pressure mode. Air drawn from the lab was used as the drying medium.

| | |
|---|---|
| Pump rate | 15% (5.4ml/min) |
| Inlet temperature | 90°C |
| Aspiration | 100% |
| N₂ flow (atomisation) Max | (approx. 55L/hr) |

### Product

A dry white powder was obtained. This material was redispersed in demineralised water at a concentration of 1mg/ml (0.1wt%, 0.01wt% chlorothalonil). This produced an opaque white dispersion. At this concentration, the material dispersed at a similar rate to example 2 (less than 30 seconds).

| | |
|---|---|
| Viscosity | 0.93cP |
| Particle size | **402nm** (diameter) |
| Standard deviation | ± 15.1nm |
| PdI | 0.228 |

### Example 4-5

### Nano-dispersion of Ketoconazole (water-insoluble fungicide) from a single phase chloroform solution stabilised with PVA/PEG (single phase method)

2g (10%) Ketoconazole and 18g (90%) poly(vinyl alcohol) (10kDa, Aldrich) were dissolved in 500ml ethanol and 360ml water. The resulting solution was spray dried at an inlet temperature of 150°C and a pump rate of approximately 3.6 ml/minute. The recovered dry white powder redispersed in water to give a clear suspension of average particle size **24.2nm** (Z-ave).

100mg (10%) Ketoconazole, 500mg (50%) poly(ethylene glycol) (10kDa, Fluka) and 400mg (40%) sodium lauryl ether sulfate (SLES) were dissolved in 60ml ethanol and 60ml water. The resulting solution was spray dried at an inlet temperature of 180°C and a pump rate of approximately 3.6 ml/minute. The recovered dry white powder redispersed in water to give a clear suspension of average particle size **16.1nm** (Z-ave).

### Example 6

### Nano-dispersion of Propiconazole (water-insoluble fungicide) from a single phase chloroform solution stabilised with SLES/PEG (single phase method)

Typically between 20mg and 800mg (between 1% and 40%) propiconazole, between 600mg and 1100mg (between 32% and 55%) poly(ethylene glycol) (10kDa, Fluka), and between 600mg and 880mg (between 28% and 44%) sodium lauryl ether sulfate (SLES) were dissolved in between 30ml and 80ml of ethanol and 30ml of water. The resulting solutions were spray dried at an inlet temperature of 180°C and an approximate pump rate of 3.6 ml/minute. The products were recovered as dry white powders.

### Examples 7-9

### Biocidal activity of Ketoconazole and Propiconazole produced in Examples 4-6

These materials produced according to Examples 4-6 were examined for their biocidal efficacy by following a standardised Minimum Inhibitory Concentration (MIC) test. The test consisted of known number of cells of a strain of *Candida albicans* (CA) in YEME (yeast extract malt extract media), inoculating concentrations of the active material and blanks contained in a 96 well plate. The plates were incubated at 37°C overnight, and then examined with a UV plate reader at a wavelength of 550nm. Concentrations of biocide that inhibited cell growth resulted in a well with very low optical density (visually clear), whereas wells in which cells growth occurred had a very high optical density (visually opaque). The MIC was defined as the lowest concentration of biocide that resulted in total inhibition of cell growth, when incubated overnight.

Equivalent experiments were also conducted to determine the MIC for the biocide active dissolved in a water miscible solvent. In these cases the "blank" reference material was simply a sample of the solvent containing no active compound.

All experiments were repeated 4 times. Results are presented in the tables below.

### Example 7: Ketoconazole:

| **Material** | **Initial concentration** | **Particle size (Z-ave, nm)** | **MIC (mg/L)** |
|---|---|---|---|
| 13/28/20 (SLES/PEG blank matrix) | equivalent to 0.15 mg/ml active | n/a | no inhibition observed |
| **As Example 5** | **0.15 mg/ml active** | **16.1** | **∼14** |
| **As Example 4** | **0.15 mg/ml active** | **24.2** | **∼38** |
| DMSO | 25%^{v}/ᵥ | n/a | ∼9.2%^{v}/ᵥ |
| PVA (blank) | equivalent to 0.15 mg/ml active | n/a | no inhibition observed |
| Ketoconazole in DMSO | 0.15 mg/ml in 25%^{v}/ᵥ solvent | n/a | 65 |

From these results it can be seen that the materials of the present invention (shown in bold in the table) were more effective at inhibiting the growth of CA in YEME than an equivalent solution of the active in DMSO. It was determined that at the concentrations involved, the MIC recorded was attributable to the active and not the solvent. It was also shown that the preparation with a smaller particle size was more active than an equivalent dispersion of larger particles. In both cases the matrix material was not found to exhibit any biocidal activity at these concentrations.

### Example 8: Propiconazole:

All experiments were conducted in YEME media against a CA culture.

| **Material Ref (14/22/..)** | **Initial concentration (mg/ml of active)** | **Loading of active (%)** | **Particle size (Z-ave, nm)** | **MIC (mg/L)** |
|---|---|---|---|---|
| 16 | 0.25 | 1 | 6.45 | 4 |
| 17 | 0.25 | 3 | 4.97 | 11 |
| 11 | 0.25 | 5 | 6.08 | 14 |
| 04 | 0.25 | 10 | 4.84 | 23 |
| 12 | 0.25 | 15 | 6.90 | 27 |
| 13 | 0.25 | 20 | 4.66 | 32 |
| 14 | 0.25 | 25 | 6.85 | 31 |
| 15 | 0.25 | 30 | 16.4 | 38 |
| 18 | 0.25 | 35 | 40.8 | 46 |
| 19 | 0.25 | 40 | 245 | 46 |

The MIC of propiconazole dissolved in a water/propylene glycol cosolvent mixture is for comparison 91.6 mg/L. In all cases it was shown that the biocidal activity of the preparations according to the present invention was much greater than that of the equivalent solution of active in water miscible solvent.

At the equivalent concentrations, no inhibition of cell growth was observed for the blank matrix material, with the exception of the very low loadings of active. In these cases, some inhibition was attributable to the matrix material, but at orders of magnitude lower than the active formulation.

The greatest efficacy was observed with material 14/22/16, which was more than 20 times more effective than the equivalent preparation of soluble active.

### Example 9:

Effect of SLES/PEG ratio in the matrix (fixed active loading) :

| **Material Ref (14/22/..)** | **Initial concentration (mg/ml of active)** | **PEG (wt.%)** | **SLES (wt.%)** | **MIC (mg/L)** |
|---|---|---|---|---|
| 34 | 0.25 | 0 | 90 | 16 |
| 28 | 0.25 | 10 | 80 | 14 |
| 29 | 0.25 | 20 | 70 | 16 |
| 30 | 0.25 | 30 | 60 | 16 |
| 31 | 0.25 | 40 | 50 | 23 |
| 04 | 0.25 | 50 | 40 | 23 |
| 32 | 0.25 | 60 | 30 | 27 |
| 33 | 0.25 | 70 | 20 | 31 |
| 35 | 0.25 | 80 | 10 | 46 |

It has also been demonstrated that biocidal activity of the materials according to the present invention is influenced by the composition of its matrix. In this case, the activity of the formulation (and hence efficacy of the active) increased 3 fold when the proportion of surfactant is increased from 10 wt.% to 80 wt.%.

### Example 10-12

### Nano-dispersion of Triclosan (water-insoluble antibacterial) from a single-phase mixture of cosolvents stabilised by SDS only.

| *Composition* | |
|---|---|
| Triclosan | 2.0g (20wt.%) |
| SDS | 8.0g (80wt.%) |
| Water miscible solvent | 125ml (50/50^{v}/ᵥ mixture) |

Three different water miscible organic solvents were employed (ethanol, acetone and isopropyl alcohol). At these concentrations, the solid components are readily soluble in the cosolvent mixture at room temperature (21.5°C).

### Drying

The solution was spray dried using a Buchi B-290 bench top spray dryer, operated in a negative pressure mode. Air drawn from the lab was used as the drying medium.

| | |
|---|---|
| Pump rate | 7% (2.5ml/min) |
| Inlet temperature | 120°C |
| Aspiration | 100% |
| N₂ flow (atomisation) | Max (approx. 55L/hr) |

### Products

For each different cosolvent system, a dry white powder was obtained. These material was redispersed in demineralised water at a concentration of 1mg/ml (0.1wt%), rapidly producing a crystal clear dispersion that remained stable for more than 12 hours. All the dispersions appeared to produce particles of the similar sizes and distributions.

Details concerning these experiments and their results are given in the table below:

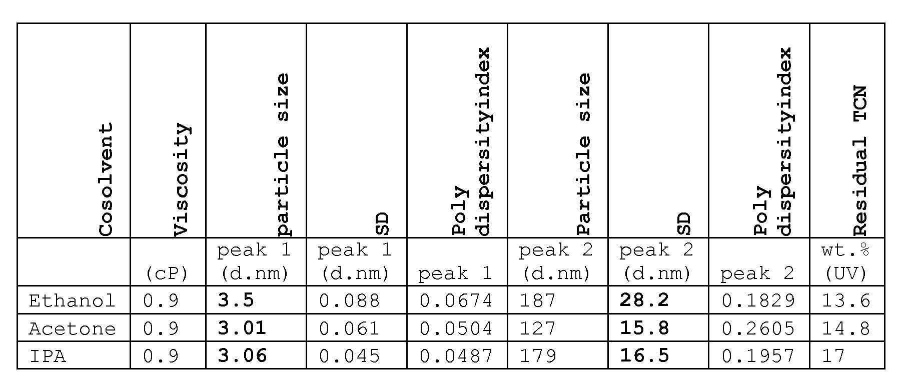

### Example 13:

### Nano-dispersion of Isoproturon (ISP - water-insoluble herbicide, emulsion method )

The formulation consisted of:

| Phase 1: | | |
|---|---|---|
| 10% ISP (28) | 500mg | in 10ml Chloroform |

| Phase 2: | | |
|---|---|---|
| 60% PVA (10kDa) | 3000mg | |
| 30% SDS | 1500mg | in 75ml water |

The two phases were continuously cooled using a water jacketed beaker whilst being emulsified. Emulsification was achieved by sonicating for 5 minutes at 50% power, then for a further 2 minutes at 100% power (using a 1kW probe type sonicator).

The resulting emulsion was spray dried at an inlet temperature of 150°C and a pump rate of 5.6 ml/min. Aspiration and atomisation gas were set to maximum.

The resulting dry white powder redispersed to give a slightly cloudy suspension at a concentration of 1 mg/ml. The particle size was measured as **297** ⁺/₋ 8.66nm.

### Example 14:

### Nano-dispersion of Isoproturon (ISP - water-insoluble herbicide ) with co-surfactant (emulsion method)

Formulation was as Example 13, but contained a small quantity of additional volatile cosurfactant (n-butanol).

| Phase 1: | | |
|---|---|---|
| 10% ISP (28) | 500mg | in 10ml Chloroform and 2ml n-butanol |

| Phase 2: | | |
|---|---|---|
| 60% PVA (10kDa) | 3000mg | |
| 30% SDS | 1500mg | in 75ml water |

The two phases were continuously cooled using a water jacketed beaker whilst being emulsified. Emulsification was achieved by sonicating for 5 minutes at 50% power, then for a further 2 minutes at 100% power (using a 1kW probe type sonicator). The resulting emulsion was spray dried at an inlet temperature of 150°C and a pump rate of 5.6 ml/min. Aspiration and atomisation gas were set to maximum.

The resulting dry white powder redispersed to give a slightly cloudy suspension at a concentration of 1 mg/ml, although noticeably clearer than that of example 13. The particle size was measured as **252** ⁺/- 14.0nm.

### Example 15-16

### Nano-dispersion of Azoxystrobin (water insoluble fungicide), single phase method

### Example 15:

1.10 g Azoxystrobin™, 2.00 g Brij^{®} 58 (Aldrich), and 6.90 g Polyvinylpyrrolidone (Mw 45,000, Aldrich) were all dissolved into 200 ml DCM. The solution was then spray dried at 70°C.

The dry powder was then dispersed into distilled water giving 1wt% AzB in dispersion and the nanoparticle size was measured with Malvern Nano-S. (SG-15)

### Example 16

5.00 g Azoxystrobin, 10.00 g Poly(ethylene glycol)-*block-*poly(propylene glycol)-*block*-poly(ethylene glycol) (Mw 8,400, Aldrich), and 35.00 g Polyvinylpyrrolidone (Mw 45,000, Aldrich) were all dissolved into 1.0 litre DCM. The solution was then spray dried at 70°C.

The dry powder was then dispersed into distilled water giving 1wt% AzB in dispersion and the nanoparticle size was measured with Malvern Nano-S. (SG-16)

Details concerning these experiments and their results are given in the table below.

| **Ex.** | **AzB, mg/ml** | **Surfactant, mg/ml** | **PVP, mg/ml** | **Solvent, ml** | **Spray dry temp., C** | **PS, nm** |
|---|---|---|---|---|---|---|
| **15** | **5.5** | **Brij 58, 10.0** | **34.5** | **DCM, 200** | **70** | **532** |
| **16** | **5.0** | **Pluronic, 10.0** | **35.0** | **DCM, 1000** | **70** | **356** |
| **Spray drying conditions:** | | | | | | |
| **Aspiration rate: 100%;** | | | | | | |
| **Pump rate: 1.80 ml/min.** | | | | | | |

### Example 17A-L

### Nano dispersion of λ-cyhalothrin (water insoluble insecticide), freeze drying, single phase method

A single phase solution of the insecticide λ-cyhalothrin, PEG and the PEG based surfactants Pluronic™ F68 and Pluronic™ F127 was prepared in chloroform. This was freeze dried using a "Christ alpha 2-4 LSC" freeze dryer in single batches on a 5ml per sample scale (i.e. 250mg solids in each sample).

Both the sample and the freeze drying shelf were pre-cooled in liquid nitrogen, to a final temperature below -140°C (operating limit of the freeze dryers temperature probes). Once the vacuum had been achieved (∼0.080 mbar) the samples remained frozen due to the self-cooling effect produced by the sample's own sublimation. Results and compositions are given in the table below:

| **Example 17** | **F68** | **F127** | **PEG** | **active** | **Particle size** |
|---|---|---|---|---|---|
| A | 0.9 | 0 | 0 | 0.1 | 66.19 |
| B | 0 | 0.9 | 0 | 0.1 | 44.24 |
| C | 0 | 0 | 0.9 | 0.1 | 168.2 |
| D | 0.7 | 0 | 0 | 0.3 | 102 |
| E | 0 | 0.7 | 0 | 0.3 | 80.99 |
| F | 0 | 0 | 0.7 | 0.3 | 240.5 |
| G | 0 | 0.4 | 0.4 | 0.2 | 94.59 |
| H | 0.4 | 0 | 0.4 | 0.2 | 127.5 |
| I | 0.3 | 0.3 | 0.3 | 0.1 | 51.87 |
| J | 0.26667 | 0.26667 | 0.2667 | 0.2 | 90.92 |
| K | 0.26667 | 0.26667 | 0.2667 | 0.2 | 93.54 |
| L | 0.26667 | 0.26667 | 0.2667 | 0.2 | 72.5 |

## Claims

1. A solvent-free fungicidal composition of improved efficacy comprising at least one water-insoluble strobilurin fungicide and a water-soluble carrier material, wherein the water-insoluble strobilurin fungicide is dispersed through the carrier material in nano-disperse form having a peak diameter of the nano-disperse form below 1000nm.

2. A composition as claimed in claim 1 wherein the strobilurin fungicide is azoxystrobin.

3. A composition according to claim 1 or claim 2 wherein the peak diameter of the water-insoluble strobilurin fungicide is below 800nm, preferably below 500nm, more preferably below 200nm and most preferably below 100nm.

4. A composition according to any preceding claim wherein the water-insoluble strobilurin fungicide has solubility in water at ambient temperature of less than 5g/L preferably of less than 1g/L, more preferably less than 100mg/L, and most preferably less than 15mg/L.

5. An aqueous dispersion of a water-insoluble strobilurin fungicide and a water-soluble carrier material obtained by the dispersion of the composition of any preceding claim in water.

6. A process for preparing a composition according to any one of claims 1-4 which comprises spray drying a solution of the strobilurin fungicide and a solution of a water-soluble carrier to obtain a solvent free dispersion of the strobilurin fungicide in the carrier, which, when dissolved in water, produces a nano-disperse strobilurin fungicide.

7. A process for preparing a composition according to any one of claims 1-4 which comprises the steps of:
a) providing an emulsion comprising:
i) a solution of the strobilurin fungicide in at least one water-immiscible solvent for the same, and
ii) an at least partially aqueous solution of the carrier material, and,
b) drying the emulsion to remove water and the water-immiscible solvent to obtain a nano-dispersion of the strobilurin fungicide in the carrier.

8. A process for preparing a composition according to any one of claims 1-4 which comprises the steps of:
a) providing a mixture comprising:
i) at least one non-aqueous solvent,
ii) optionally, water,
iii) a water-soluble carrier material soluble in the mixture of (i) and (ii) and
iv) a water-insoluble strobilurin fungicide which is soluble in the mixture of (i) and (ii), and,
b) drying said mixture to remove the non-aqueous solvent and any water present to obtain a nano-dispersion of the strobilurin fungicide in the carrier.

9. A process for the preparation of a fungicidal composition for use in the prophylaxis or treatment of infections or infestations which comprises the step of preparing a composition according to any one of claims 1-4.

10. A composition obtainable by the process of any one of claims 6-9.

11. A process for the treatment of a substrate other than a medical treatment which comprises the step of contacting the substrate with a composition according to any one of claims 1-4 or 10.

12. A process for the delivery of a water-insoluble strobilurin fungicide which comprises the steps of:
a) dissolving in water a nano-dispersion of a water-insoluble strobilurin fungicide in a water soluble carrier material, according to any one of claims 1-4 or 10,
b) optionally, adding other components to the dispersion, and,
c) treating a substrate with the aqueous nano-dispersion of the strobilurin fungicide.

13. A process according to claim 12 wherein step (c) comprises spraying, dipping or washing the substrate.

14. A process according to claim 12 or 13 wherein the substrate is selected from a plant, soil, an animal, bedding for animals, fodder or an article manufactured from a plant or an animal.

15. Use of a composition according to any one of claims 1-4 or 10 for the treatment of an infestation.

16. A process according to claims 7-9 wherein the drying step comprises spray drying.
